# EUROPEAN PATENT SPECIFICATION

(11) **EP 2 556 816 B1**
(45) Date of publication and mention of the grant of the patent: **26.06.2019**
(21) Application number: 12472001.2
(22) Date of filing: 23.03.2012
(51) Int. Cl.: A61K 8/04, A61K 8/19, A61K 8/22, A61K 8/41, A61K 8/44, A61K 8/64, A61K 8/73, A61K 8/81, A61K 8/893, A61Q 5/10

(54) **Permanent hair dye in the form of foam**
Permanente Haarfärbung in Form eines Schaums
Colorant capillaire permanent sous la forme d'une mousse

(30) Priority: 30.03.2011 BG 11090311
(43) Date of publication of application: 13.02.2013
(73) Proprietor: "Aroma" Plc., 1271 Sofia (BG)
(72) Inventor: Ivanov, Ivan Vasilev, 1582 Sofia (BG)

(56) References cited:
- US-A1- 2010 313 362
- Anonymous: "Home Coloring Kit - PERFECT HAIR- EVERY TIME", Miracles in the city , 3 February 2009 (2009-02-03), XP002712340, Retrieved from the Internet: URL:http://miraclesinthecity.com/new-store .html?id=501 [retrieved on 2013-09-05]
- DATABASE GNPD [Online] MINTEL; 1 October 2009 (2009-10-01), "Permanent hair colourant", XP002712341, Database accession no. 1189998
- DATABASE GNPD [Online] MINTEL; 2 July 2007 (2007-07-02), "Hair colourant", XP002712342, Database accession no. 740821
- DATABASE GNPD MINTEL; 1 September 2010 (2010-09-01), "Permanent Cream Hair Colour", XP002712343, Database accession no. 1398627
- DATABASE GNPD [Online] MINTEL; 1 April 2011 (2011-04-01), "Hair Colour", XP002712344, Database accession no. 1536026

## Description

### Field of the Invention:

The invention in focus is concerning a permanent hair dye in the form of foam, formulated in such a way to protect the hair and make it possible for an even hair-coverage through all its length.

### Background of the Invention:

The conventional hair dyes are either liquids or emulsions (cream-like mass). These conventional hair dyes' disadvantage is the difficult application on the hair so that a permanent and even colour is achieved. Usually it is recommended that these colorants are applied from the hair's roots to its tips.
Colouring shampoos are also commonly known products for hair dyeing. The foam achieved with application of these products is insufficiently stable to reach permanent colouring. The achieved colour fades in two to three washes.

A two-component composition in the form of foam as a cosmetic product is well-known and also described in EP 2258338 (A1) and WO 20099144952 (A1). The surfactants used to reach stable and high-quality foam are in concentrations different than the ones used in the current invention.

The invention in focus aims: to achieve a composition for permanent hair dye in the form of foam that (foam) is stable during colouring and does not disintegrates by the body heat; to ensure even and smooth coverage through the whole length of the hair so that the achieved colours are deep, without any nuances. This is accomplished by a specially selected combination of different types of surfactants, foam stabilizers, and active ingredients.

### DETAILED DESCRIPTION OF THE INVENTION

The invention at focus is a permanent hair dye under the form of foam by mixing together minimum two liquid components, (i) and (ii), component (i) containing dyeing agent, and component (ii) containing oxidative agent. Component (i) contains a composition that has PH of 9,5 - 11,0 and the composition is of the following ingredients: hydrolised silk extract as an active ingredient, polymeric alkyl / allyl quaternary ammonium acrylate and hydrolysed wheat protein as an conditioning agent with in range from 3.0 % to 7.0 % by weight, (D) water-soluble silicone acting as a stabilizer of the foam in range from 0.05 % to 5.0 % by weight, (B) alkali metallic ion in mol/kg from 0,05 to 0,19 mol/kg, (C1) oxidative dyes with phenol hydroxyl group (C2) anionic surfactants (E) non-ionic surfactants and (F) amphoteric surfactants wherein the total concentration of (C1 + C2) in mol/kg from 0,015 to 0,155 mol/kg, and the total concentration of (E + F + C2) in mol/kg from 0,16 to 0,80.

Component (ii) has a PH from 2, 5 to 4, 0 and contains hydrogen peroxide from 3.0 % to 12.0 %.

### Surfactant combination:

Methyl taurates of fatty acids, and specifically Sodium Methyl Cocoyl Taurate of coconut oil (Hostapon™) from 0,005 to 0,040 mol/kg, are used as anionic surfactants (C2).

These surfactants are also a source of alkali metallic ion. The non-ionic surfactants (E) used are in the polysorbate group, and more specifically in the polyoxiethylene-sorbitan monosulfate. It is used in combination with polyglucosides in concentration from 0,135 to 0,690 mol/kg.

As a preferred amphoteric foamer (F) is used betaine in concentration from 0,02 to 0,07 mol/kg.

### Foam stabilizers:

The foam stabilizers that could be used are various water-soluble polyether-modified silicones in concentration from 0.01 % to 10 % by weight. The foam stabilizer used (D) is PEG -12 Dimethicone in concentration from 0.05 % to 5.0 % by weight. It forms a stable foam that suits well with the hair's keratin.

The selected combination of anionic surfactants (C2), amphoteric surfactants (F), non-ionic surfactants, and foam stabilizers (D) both increases the quality and the volume of the foam and makes it stable during the 20-30 minutes application, averting the foam from being destructured by the body's heat (around 36° C).

### Active ingredient:

Hydrolysed silk extract is used. The silk extract is a protein containing 18 amino acids, some of them being glycine, alanine, seryne and tyrosine. These amino acids are of crucial importance for healthy hair and skin. The silk extract has excellent hygroscopic properties that enhance the hair's elasticity.

### Conditioning agent

The conditioning agent used is polymeric alkyl / allyl quaternary ammonium acrylate and hydrolysed wheat protein (Lowenol PWW ™). It is a cationic polyquaternium agent with good sustenance to the hairs. It has a good compatibility with the non-ionic, anionic, and amphoteric surfactants. It lowers the electrostatic effect and restrains the hair from scattering, making it soft, smooth and shiny.

### Oxidative dyes with phenol hydroxyl group

The utilized dyes (C1) are oxidation dyes, having a phenolic OH - group. Some of the specific dyes used are p-aminophenol, m-aminophenol, 2-methyl-5-hydroxy-ethyl-aminophenol,4-amino-2-hydroxytoluene, 5-amino-6-chloro-o-cresol, 2 - methylresorcinol, 4-chlororesorcinol.

### Alkali metallic ions

The alkali metallic ions' purpose is to make the hairs swell and to give more saturated shades. The following agents are used as a source of the alkali metallic ions: tetrasodium EDTA and its acids, sodium metabisulfite, sodium chloride, and sodium hydroxide. Besides a source of alkali metallic ions, these agents have additional functions. Tetrasodium EDTA and its acids, for example, is essentially a chelate agent, sodium metabisulfite - deoxidizer, sodium chloride (used as a regulator of the consistency / thickener), potassium hydroxide (used as a buffering agent).

### Alkali agents

As the alkali agents are used ammonium hydroxide, potassium hydroxide, triethanolamine and aminomethyl propanol. The first three alkaline agents are used preferably in ammonia hair dyes (hair colorans) while aminomethyl propanol is used mainly in non - amonia hair dyes.

### Examples:

**Table No1 - Non - ammonia hair dye (middle brown)**

| | **INGREDIENTS:** | **FUNCTION** | Molecular weight (g/mol) | PH for 100 g | As 100 % active ingredient |
|---|---|---|---|---|---|
| **COMPONENT (i)** | | | | | |
| **E** | POLYGLUCOSIDE | MILD NONIONIC SURFACTANT | 320.42 | 4.0 | 2.120 |
| **F** | BETAINE | AMPHOTERIC SURFACTANT | 342.52 | 1.5 | 0.570 |
| **E** | MONOLAURATE OF POLYOXYETHYLENATED SORBITAN | NONIONIC SURFACTANT | 1227.5 | 3.5 | 3.395 |
| | **SUM (E + F + C2) % =** | **Range from 5.0 % to 30.0 %** | | | **6.265** |
| | PROPYLENE GLYCOL | | | 4.0 | |
| **D** | POLYETHILENE GLYCOL 12 DIMETHICONE | FOAM STABILIZER - range from 0,05 % to 5.0 % by weight | | 3.0 | |
| **B** | SODIUM METHABISULFITE | Alkali metal ion | 190.11 | 0.5 | 0.146 |
| **B** | TETRASODIUM EDTA | | 380.17 | 0.3 | 0.073 |
| **B** | SODIUM CHLORIDE | | 58.44 | 0.35 | 0.394 |
| **B** | POTASSIUM HYDROXIDE | | 56.106 | 0.35 | 0.279 |
| | **SUM (B) % =** | **Range from 0.45 % to 1.60 %** | | | **0.625** |
| **B,C2** | SODIUM METHYL COCOYL TAURATE OF COCONUT OIL | Alkali metal ion and anionic surfactant | 161.16 | 0.6 | 0.180 |
| **C1** | p-AMINOPHENOL | OXIDATION DYE, HAVING A PHENOLIC OH-GROUP | 109.13 | 0.3 | 0.028 |
| **C1** | m-AMINOPHENOL | | 109.13 | 0.225 | 0.02 |
| **C1** | 4-AMINO-2-HYDROXYTOLUENE | | 123.15 | | |
| **C1** | 5-AMINO -6-CHLORO-O-CRESOL | | 157.65 | | |
| **C1** | RESORCINOL | | 110.1 | 0.62 | 0.620 |
| **C1** | 4- CHLORORESORCINOL | | 144.56 | | |
| **C1** | 2-METHIL-5 - HIDROXYEHILAMINOPHENOL | | 167.21 | | |
| **C1** | 2-METHILRESORCINOL | | 124.17 | | |
| **SUM (C1) % =** | | | | | **1.145** |
| **SUM (C1 + C2) % =** | | **Range from 0.3 % to 3.0 %** | | | **1.325** |
| | P-PHENYLENEDIAMINE | | | 0.22 | |
| | TOLUENE -2,5-DIAMINE SULPHATE | | | 0.12 | |
| | ASCORBIC ACID | | | 0.4 | |
| | **AMINOMETHYL PROPANOL** | **Alkali agent** | | 2.0 | |
| | POLYMERIC ALKYL / ALLYL QUATERNARY AMMONIUM ACRYLATE (AND) HYDROLYZED WHEAT PROTEIN | Conditioning agent - range from 3.0 % to 7.0 % by weight | | 5.0 | |
| | **Silk Extract** | **Activ Ingredient** | | 1.0 | |
| | PARFUM | | | 0.3 | |
| | Purified Water | | | 72.26 | |
| | **SUM** | | | 100 | |

**Table No 2 - Hair dye with ammonia (middle brown)**

| | **INGREDIENTS:** | **FUNCTION** | Molecular weight (g/mol) | PH for 100 g | As 100 % active ingredient |
|---|---|---|---|---|---|
| **E** | POLYGLUCOSIDE | MILD NONIONIC SURFACTANT | 320.42 | 4.0 | 2.120 |
| **F** | BETAINE | AMPHOTERIC SURFACTANT | 342.52 | 1.5 | 0.570 |
| **E** | MONOLAURATE OF POLYOXYETHYLENATED SORBITAN | NONIONIC SURFACTANT | 1227.5 | 3.5 | 3.395 |
| | **SUM (E + F + C2) % =** | **Range from 5.0 % to 30.0 %** | | | **6.265** |
| | PROPYLENE GLYCOL | | | 4.0 | |
| **D** | POLYETHILENE GLYCOL 12 DIMETHICONE | FOAM STABILIZER - range from 0,05 % to 5.0 % by weight | | 3.0 | |
| **B** | SODIUM METHABISULFITE | Alkali metal ion | 190.11 | 0.6 | 0.145 |
| **B** | TETRASODIUM EDTA | | 380.17 | 0.30 | 0.073 |
| **B** | SODIUM CHLORIDE | | 58.44 | 0.35 | 0.138 |
| **B** | POTASSIUM HYDROXIDE | | 56.106 | 0.20 | 0.139 |
| | **SUM (B) % =** | **Range from 0.45 % to 1.60 %** | | | **0.521** |
| **B,C2** | SODIUM METHYL COCOYL TAURATE OF COCONUT OIL | Alkali metal ion and anionic surfactant | 363 | 0.60 | 0.180 |
| **C1** | para-AMINOPHENOL | OXIDATION DYE, HAVING A PHENOLIC OH-GROUP | 109.13 | 0.30 | 0.300 |
| **C1** | meta-AMINOPHENOL | | 109.13 | 0.225 | 0.225 |
| **C1** | 4-AMINO-2-HYDROXYTOLUENE | | 123.15 | | |
| **C1** | 5-AMINO -6-CHLORO-O-CRESOL | | 157.65 | | |
| **C1** | RESORCINOL | | 110.1 | 0.62 | 0.620 |
| **C1** | 4- CHLORORESORCINOL | | 144.56 | | |
| **C1** | 2-METHIL-5 - HIDROXYEHILAMINOPHENOL | | 167.21 | | |
| **C1** | 2-METHILRESORCINOL | | 124.17 | | |
| **SUM (C1) % =** | | | | | **1.145** |
| **SUM (C1 + C2) % =** | | **Range from 0.3 % to 3.0 %** | | | **1.325** |
| | P-PHENYLENEDIAMINE | | | 0.22 | |
| | TOLUENE -2,5-DIAMINE SULPHATE | | | 0.12 | |
| | ASCORBIC ACID | | | 0.50 | |
| | **TRIEHANOLAMINE** | **ALKALI AGENT** | | 1.50 | |
| | **AMMONIA - 25 %** | **ALKALI AGENT** | | 7.0 | |
| | POLYMERIC ALKYL / ALLYL QUATERNARY AMMONIUM ACRYLATE (AND) HYDROLYZED WHEAT PROTEIN | Conditioning agent - range from 3.0 % to 7.0 % by weight | | 5.0 | |
| | **Silk Extract** | **Activ Ingredient** | | **1.0** | |
| | PARFUM | | | 0.5 | |
| | Purified Water | | | 65.26 | |
| | **SUM** | | | 100 | |

| **COMPONENT (ii)** | | | | | |
|---|---|---|---|---|---|
| | Hydrogen peroxide - liquid solution | Oxidative agent | | | 6% |

**Table No 3 - Non - ammonia hair dye (Dark blond)**

| | **INGREDIENTS:** | **FUNCTION** | Molecular weight (g/mol) | PH for 100 g | As 100 % active ingredient |
|---|---|---|---|---|---|
| **COMPONENT (i)** | | | | | |
| **E** | POLYGLUCOSIDE | MILD NONIONIC SURFACTANT | 320.42 | 4.0 | 2.120 |
| **F** | BETAINE | AMPHOTERIC SURFACTANT | 342.52 | 1.5 | 0.570 |
| **E** | MONOLAURATE OF POLYOXYETHYLENATED SORBITAN | NONIONIC SURFACTANT | 1227.5 | 3.5 | 3.395 |
| | **SUM (E + F + C2) % =** | **Range from 5.0 % to 30.0 %** | | | **6.265** |
| | PROPYLENE GLYCOL | | | 4.0 | |
| **D** | POLYETHILENE GLYCOL 12 DIMETHICONE | FOAM STABILIZER - range from 0,05 % to 5.0 % by weight | | 3.0 | |
| **B** | SODIUM METHABISULFITE | Alkali metal ion | 190.11 | 0.5 | 0.121 |
| **B** | TETRASODIUM EDTA | | 380.17 | 0.30 | 0.073 |
| **B** | SODIUM CHLORIDE | | 58.44 | 0.35 | 0.138 |
| **B** | POTASSIUM HYDROXIDE | | 56.106 | 0.35 | 0,244 |
| | **SUM (B) % =** | **Range from 0.45 % to 1.60 %** | | | **0.601** |
| **B,C2** | SODIUM METHYL COCOYL TAURATE OF COCONUT OIL | Alkali metal ion and anionic surfactant | 161.16 | 0.60 | 0.180 |
| **1** | p-AMINOPHENOL | OXIDATION DYE, HAVING A PHENOLIC OH-GROUP | 109.13 | | |
| **C1** | m-AMINOPHENOL | | 109.13 | | |
| **C1** | 4-AMINO-2-HYDROXYTOLUENE | | 123.15 | | |
| **C1** | 5-AMINO-6-CHLORO-O-CRESOL | | 157.65 | | |
| **C1** | RESORCINOL | | 110.1 | 0.03 | 0.030 |
| **C1** | 4-CHLORORESORCINOL | | 144.56 | | |
| **C1** | 2-METHIL-5 - HIDROXYEHILAMINOPHENOL | | 167.21 | | |
| **C1** | 2-METHILRESORCINOL | | 124.17 | 0.16 | 0.160 |
| **SUM (C1) % =** | | | | | **0.190** |
| **SUM (C1 + C2) % =** | | **Range from 0.3 % to 3.0 %** | | | **0.370** |
| | P-PHENYLENEDIAMINE | | | 0.11 | |
| | TOLUENE -2,5-DIAMINE SULPHATE | | | 0.35 | |
| | ASCORBIC ACID | | | 0.30 | |
| | **AMINOMETHYL PROPANOL** | **Alkali agent** | | 2.00 | |
| | POLYMERIC ALKYL / ALLYL QUATERNARY AMMONIUM ACRYLATE (AND) HYDROLYZED WHEAT PROTEIN | Conditioning agent - range from 3.0 % to 7.0 % by weight | | 5.0 | |
| | **Silk Extract** | **Activ Ingredient** | | **1.0** | |
| | PARFUM | | | 0.30 | |
| | Purified Water | | | 72.65 | |
| | **SUM** | | | 100 | |

**Table No 4 - Hair dye with ammonia (Dark blond)**

| | **INGREDIENTS:** | **FUNCTION** | Molecular weight (g/mol) | PH for 100 g | As 100 % active ingredient |
|---|---|---|---|---|---|
| **E** | POL YGLUCOSIDE | MILD NONIONIC SURFACTANT | 320.42 | 4.0 | 2,120 |
| **F** | BETAINE | AMPHOTERIC SURFACTANT | 342.52 | 1.5 | 0.570 |
| **E** | MONOLAURATE OF POLYOXYETHYLENATED SORBITAN | NONIONIC SURFACTANT | 1227.5 | 3.5 | 3.395 |
| | **SUM (E + F + C2) % =** | **Range from 5.0 % to 30.0 %** | | | **6.265** |
| | PROPYLENE GLYCOL | | | 4.0 | |
| **D** | POLYETHILENE GLYCOL 12 DIMETHICONE | FOAM STABILIZER - range from 0,05 % to 5.0 % by weight | | 3.0 | |
| **B** | SODIUM METHABISULFITE | Alkali metal ion | 190.11 | 0.5 | 0.121 |
| **B** | TETRASODIUM EDTA | | 380.17 | 0.3 | 0.073 |
| **B** | SODIUM CHLORIDE | | 58.44 | 0.35 | 0.138 |
| **B** | POTASSIUM HYDROXIDE | | 56.106 | 0.2 | 0.134 |
| | **SUM (B) % =** | **Range from 0.45 % to 1.60 %** | | | **0.496** |
| **B,C2** | SODIUM METHYL COCOYL TAURATE OF COCONUT OIL | Alkali metal ion and anionic surfactant | 363 | 0.6 | 0.180 |
| **C1** | p- AMINOPHENOL | OXIDATION DYE, HAVING A PHENOLIC OH-GROUP | 109.13 | | |
| **C1** | m- AMINOPHENOL | | 109.13 | | |
| **C1** | 4-AMINO-2-HYDROXYTOLUENE | | 123.15 | | |
| **C1** | 5-AMINO -6-CHLORO-O-CRESOL | | 157.65 | | |
| **C1** | RESORCINOL | | 110.1 | 0.03 | 0.030 |
| **C1** | 4- CHLORORESORCINOL | | 144.56 | | |
| **C1** | 2-METHIL-5 - HIDROXYEHILAMINOPHENOL | | 167.21 | | |
| **C1** | 2-METHILRESORCINOL | | 124.17 | 0.16 | 0.016 |
| **SUM (C1) % =** | | | | | **0.190** |
| **SUM (C1 + C2) % =** | | **Range from 0.3 % to 3.0 %** | | | **0.370** |
| | P-PHENYLENEDIAMINE | | | 0.11 | |
| | TOLUENE -2,5-DIAMINE SULPHATE | | | 0.35 | |
| | ASCORBIC ACID | | | 0.3 | |
| | **TRIEHANOLAMINE** | **ALKALI AGENT** | | 1.5 | |
| | **AMMONIA - 25 %** | **ALKALI AGENT** | | 7.0 | |
| | POLYMERIC ALKYL / ALLYL QUATERNARY AMMONIUM ACRYLATE (AND) HYDROLYZED WHEAT PROTEIN | Conditioning agent - range from 3.0 % to 7.0 % by weight | | 5.0 | |
| | **Silk Extract** | **Activ Ingredient** | | 1.0 | |
| | PARFUM | | | 0.5 | |
| | Purified Water | | | 66.1 | |
| | **SUM** | | | 100 | |

| **COMPONENT (ii)** | | | | | |
|---|---|---|---|---|---|
| | Hydrogen peroxide - liquid solution | Oxidative agent - | | | 6% |

**Table No 5 - Non - ammonia hair dye (Light blond)**

| | **INGREDIENTS:** | **FUNCTION** | Molecular weight (g/mol) | PH for 100 g | As 100 % active ingredient |
|---|---|---|---|---|---|
| **COMPONENT (i)** | | | | | |
| E | POLYGLUCOSIDE | MILD NONIONIC SURFACTANT | 320.42 | 4.0 | 2.120 |
| **F** | BETAINE | AMPHOTERIC SURFACTANT | 342.52 | 1.5 | 0.570 |
| **E** | MONOLAURATE OF POLYOXYETHYLENATED SORBITAN | NONIONIC SURFACTANT | 1227.5 | 3.5 | 3.395 |
| | **SUM (E + F + C2) % =** | **Range from 5.0 % to 30.0 %** | | | **6.265** |
| | PROPYLENE GLYCOL | | | 4.0 | |
| **D** | POLYETHILENE GLYCOL 12 DIMETHICONE | FOAM STABILIZER - range from 0,05 % to 5.0 % by weight | | 3.0 | |
| **B** | SODIUM METHABISULFITE | Alkali metal ion | 190.11 | 0.4 | 0,097 |
| **B** | TETRASODIUM EDTA | | 380.17 | 0.3 | 0.073 |
| **B** | SODIUM CHLORIDE | | 58.44 | 0.35 | 0.138 |
| **B** | POTASSIUM HYDROXIDE | | 56.106 | 0.4 | 0.279 |
| | **SUM (B) % =** | **Range from 0.45 % to 1.60 %** | | | **0.612** |
| **B,C2** | SODIUM METHYL COCOYL TAURATE OF COCONUT OIL | Alkali metal ion and anionic surfactant | 363 | 0.6 | 0.180 |
| **C1** | p- AMINOPHENOL | OXIDATION DYE, HAVING A PHENOLIC OH-GROUP | 109.13 | 0.1 | 0.100 |
| **C1** | m-AMINOPHENOL | | 109.13 | 0.05 | 0.05 |
| **C1** | 4-AMINO-2-HYDROXYTOLUENE | | 123.15 | | |
| **C1** | 5-AMINO -6-CHLORO-O-CRESOL | | 157.65 | | |
| **C1** | RESORCINOL | | 110.1 | | |
| **C1** | 4- CHLORORESORCINOL | | 144.56 | | |
| **C1** | 2-METHIL-5 - HIDROXYEHILAMINOPHENOL | | 167.21 | | |
| **C1** | 2-METHILRESORCINOL | | 124.17 | 0.2 | 0.200 |
| **SUM (C1) % =** | | | | | **0.350** |
| **SUM (C1 + C2) % =** | | **Range from 0.3 % to 3.0 %** | | | **0.530** |
| | P-PHENYLENEDIAMINE | | | 0.07 | |
| | TOLUENE -2,5-DIAMINE SULPHATE | | | 0.17 | |
| | ASCORBIC ACID | | | 0.3 | |
| | **AMINOMETHYL PROPANOL** | **Alkali agent** | | 2.0 | |
| | POLYMERIC ALKYL / ALLYL QUATERNARY AMMONIUM ACRYLATE (AND) HYDROLYZED WHEAT PROTEIN | Conditioning agent - range from 3.0 % to 7.0 % bv weight | | 5.0 | |
| | **Silk Extract** | **Activ Ingredient** | | **1.0** | |
| | PARFUM | | | 0.3 | |
| | Purified Water | | | 72.76 | |
| | **SUM** | | | 100 | |

**Table No 6 - Hair dye with ammonia (Light blond)**

| | **INGREDIENTS:** | **FUNCTION** | Molecu lar weight (g/mol) | PH for 100 g | As 100 % active ingredient |
|---|---|---|---|---|---|
| **E** | POLYGLUCOSIDE | MILD NONIONIC SURFACTANT | 320.42 | 4.0 | 2.120 |
| **F** | BETAINE | AMPHOTERIC SURFACTANT | 342:52 | 1.5 | 0.570 |
| **E** | MONOLAURATE OF POLYOXYETHYLENATED SORBITAN | NONIONIC SURFACTANT | 1227.5 | 3.5 | 3.395 |
| | **SUM (E + F + C2) % =** | **Range from 5.0 % to 30.0 %** | | | **6.265** |
| | PROPYLENE GLYCOL | | | 4.0 | |
| **D** | POLYETHILENE GLYCOL 12 DIMETHICONE | FOAM STABILIZER - range from 0,05 % to 5.0 % by weight | | 3.0 | |
| **B** | SODIUM METHABISULFITE | Alkali metal ion | 190.11 | 0.4 | 0.097 |
| **B** | TETRASODIUM EDTA | | 380.17 | 0.3 | 0.0739 |
| **B** | SODIUM CHLORIDE | | 58.44 | 0.35 | 0.138 |
| **B** | POTASSIUM HYDROXIDE | | 56.106 | 0.4 | 0.209 |
| | **SUM (B) % =** | **Range from 0.45 % to 1.60 %** | | | **0.542** |
| **B,C2** | SODIUM METHYL COCOYL TAURATE OF COCONUT OIL | Alkali metal ion and anionic surfactant | 363 | 0.6 | 0.180 |
| **C1** | p-AMINOPHENOL | OXIDATION DYE, HAVING A PHENOLIC OH-GROUP | 109.13 | 0.1 | 0.10 |
| **C1** | m- AMINOPHENOL | | 109.13 | 0.05 | 0.050 |
| **C1** | 4-AMINO-2-HYDROXYTOLUENE | | 123.15 | | |
| **C1** | 5-AMINO -6-CHLORO-O-CRESOL | | 157.65 | | |
| **C1** | RESORCINOL | | 110.1 | | |
| **C1** | 4- CHLORORESORCINOL | | 144.56 | | |
| **C1** | 2-METHIL-5 - HIDROXYEHILAMINOPHENOL | | 167.21 | | |
| **C1** | 2-METHILRESORCINOL | | 124.17 | 0.2 | 0.200 |
| **SUM (C1) % =** | | | | | **0.350** |
| **(C1 + C2) % =** | | **Range from 0.3 % to 3.0 %** | | | **0.530** |
| | P-PHENYLENEDIAMINE | | | 0.07 | |
| | TOLUENE -2,5-DIAMINE SULPHATE | | | 0.17 | |
| | ASCORBIC ACID | | | 0.3 | |
| | **TRIEHANOLAMINE** | **ALKALI AGENT** | | **1.5** | |
| | **AMMONIA - 25 %** | **ALKALI AGENT** | | **8.0** | |
| | POLYMERIC ALKYL / ALLYL QUATERNARY AMMONIUM ACRYLATE (AND) HYDROLYZED WHEAT PROTEIN | Conditioning agent - range from 3.0 to 7.0 % by weight | | 5.0 | |
| | **Silk Extract** | **Activ Ingredient** | | **1.0** | |
| | PARFUM | | | 0.5 | |
| | Purified Water | | | 65.16 | |
| | **SUM** | | | 100 | |

| **COMPONENT (ii)** | | | | | |
|---|---|---|---|---|---|
| | Hydrogen peroxide - liquid solution | Oxidative agent | | | 6% |

## Claims

1. Permanent hair dye under the form of foam obtained by mixing together minimum two liquid components, (i) and (ii), component (i) containing dyeing agent, and component (ii) containing oxidative agent, component (i) contains a composition that has PH of 9,5 - 11,0 and the composition is of the following ingredients: hydrolized silk extract as an active ingredient, polymeric alkyl / allyl quaternary ammonium acrylate and hydrolysed wheat protein as a conditioning agent with in range from 3.0 % to 7.0 % by weight, (D) water-soluble silicone acting as a stabilizer of the foam in range from 0.05 % to 5.0 % by weight, (B) alkali metallic ion in mol/kg from 0,05 to 0,19 mol/kg (C1) oxidative dyes with phenol hydroxyl group, (C2) anionic surfactants (E) non-ionic surfactants and (F) amphoteric surfactants wherein the total concentration of (C1 + C2) in mol/kg from 0,015 to 0,155 mol/kg, and the total concentration of (E + F + C2) in mol/kg from 0,16 to 0,80. Component (ii) has a PH from 2, 5 to 4, 0 and contains hydrogen peroxide from 3.0 % to 12.0 %.

2. Permanent hair dye, in accordance with claim 1, **characterized by** the facts that the anionic surfactants (C2) are sodium methyl cocoyl taurate of coconut oil.

3. Permanent hair dye, in accordance with claim 1, where the liquid compositions (i) and (ii) are mixed in a non-aerosol container with a mechanic pump.

## Patentansprüche

1. Permanente Haarfarbe in Form eines Schaums, der durch Mischung von mindestens zwei flüssigen Komponenten (i) und (ii) hergestellt ist, unter welchen die Komponente (i) Farbstoff beinhaltet, und die Komponente (ii) Oxidationsmittel beinhaltet, welche Haarfarbe **dadurch sich kennzeichnet, dass** die Komponente (i) eine Zusammensetzung mit pH 9,5 - 11,0 aus folgenden Bestandsteilen ist: Seidenextrakt als aktiven Bestandsteil, Polymerakril/-alil, Quarternärammoniumakrylat und hydrolysierten Getreideprotein in Grenzen ab 3 bis 7 Massenprozenten als Konditionierungsagent, (D) wasserlöslichen Silicon als Stabilisierungsmittel des Schaums in Grenzen ab 0,05 bis 5 Massenprozenten, (B) alkalisches Metallion in Grenzen ab 0,05 bis 0,19 mol/kg, (C1) Oxidfarbstoffe mit Phenolhydroxylgruppe, (C2) anione oberflächenaktive Stoffe, (E) nichtione oberflächenaktive Stoffe und (F) amphotere oberflächenaktive Stoffe, wobei die Gesamtkonzentration (C1 + C2) mol/kg in den Grenzen 0,015 bis 0,155 mol/kg steht, und die Gesamtkonzentration (E + F + C2) mol/kg in den Grenzen 0,016 bis 0,80 mol/kg steht.
Die Komponente (ii) hat pH in den Grenzen 2,5 - 4,0 und beinhaltet einen Anteil vom Wasserstoffperoxid zwischen 3 % und 12 %.

2. Permanente Haarfarbe laut des Anspruchs 1, **dadurch gekennzeichnet, dass** die anione oberflächenaktive Stoffe (C2) Natriummethylcocoyl, Thauride des Kokosöls sind.

3. Permanente Haarfarbe laut des Anspruchs 1, bei welcher die flüssigen Komponentem (i) und (II) in einem Flakon ohne Aerosol gemisch werden, der mit mechanischer Pumpe ausgerüstet ist.

## Revendications

1. Coloration capillaire permanente sous forme de mousse obtenue en mélangeant au moins deux composants aqueux (i) et (ii) dont le composant (i) contient un agent colorant et le composant (ii) contient un agent oxydant **se caractérisant par le fait que** le composant (i), représentant une composition ayant un pH compris entre 9,5-11,5 contient les ingrédients suivants: extrait de soie hydrolysée en tant qu'ingrédient actif, alkyle polymérique/acrylate d'ammonium quaternaire d'allyle et protéines de blé hydrolysées comme conditionneur allant de 3 à 7 % en poids, (D) silicone hydrosoluble agissant comme stabilisateur de la mousse allant de 0,05 à 5 % en poids, (B) ion métallique alcalin allant de 0,05 à 0,19 mol/kg, (C1) colorants oxydants avec groupe hydroxyle phénolique, (C2) tensioactifs anioniques, (E) tensioactifs non ioniques, (F) tensioactifs amphotères dans lesquels la concentration totale (C1 + C2) dans un mol/kg est comprise entre 0,015 et 0,155 mol/kg et la concentration totale (E+F+C2) dans un mol/kg est de 0,16 à 0,80.
Le composant (ii) a un pH de 2,5 à 4,0 et contient du peroxyde d'hydrogène de 3,0 à 12 %.

2. Coloration capillaire permanente selon la revendication 1 **se caractérisant par le fait que** les tensioactifs anioniques (C2) sont du sodium n-méthyle-n-cocoyl taurate de l'huile de coco.

3. Coloration capillaire permanente selon la revendication 1 où les composants aqueux (i) et (ii) sont mélangés dans un flacon non aérosol muni d'une pompe mécanique.
